# EUROPÄISCHE PATENTSCHRIFT

(11) **EP 0 612 840 B1**
(45) Veröffentlichungstag und Bekanntmachung des Hinweises auf die Patenterteilung: **06.05.1999**
(21) Anmeldenummer: 94102385.5
(22) Anmeldetag: 17.02.1994
(51) Int. Cl.: C11B 9/00, A61K 7/46, C07C 35/08

(54) **Cyclische Verbindungen und ihre Anwendung als Riechstoffe**
Cyclic compounds and their use as perfuming ingredients
Composés cycliques et leur usage comme ingredients parfumants

(30) Priorität: 24.02.1993 CH 567/93
(43) Veröffentlichungstag der Anmeldung: 31.08.1994
(73) Patentinhaber: GIVAUDAN-ROURE (INTERNATIONAL) S.A., CH-1214 Vernier (CH)
(72) Erfinder: Etzweiler, Franz, CH-8606 Greifensee (CH); Helmlinger, Daniel, CH-8600 Dübendorf (CH)
(74) Vertreter: Urech, Peter, Dr.

(56) Entgegenhaltungen:
- EP-A- 0 258 787
- EP-A- 0 427 965
- FR-A- 2 300 752
- GB-A- 865 819
- US-A- 4 757 051
- S.ARCTANDER 'Perfume and Flavor Chemicals' 1969 , S.ARCTANDER , MONTCLAIR,N.J. band I *Nr.:432,433,435,436,438,439,440,441,442*

## Beschreibung

Die vorliegende Erfindung betrifft neue Riechstoffkompositionen, die durch einen Gehalt an cyclischen Verbindungen der allgemeinen Formel 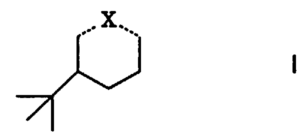 worin ...X... eine der Bedeutungen
a) >C=O
b) >CH-OR
worin R H, COCH₃, COCH₂CH₃, CO-CH(CH₃)₂, CO-CH=CH₂, COOCH₃ ist
wobei das Verhältnis cis/trans in den Isomeren von I 65-95:35-5 beträgt,
c) >C=N-OH
d) 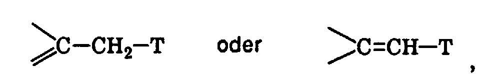 wobei T = CN oder CHO
e) 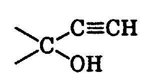
f) 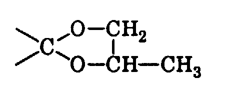 oder
g) 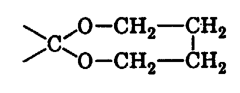 hat,
gekennzeichnet sind.

Aus dieser Gruppe von Verbindungen sind die Verbindungen I' mit den Bedeutungen b), d), f) und g) neue Verbindungen. Die restlichen Verbindungen der Formel I sind zwar zum Teil bekannt, jedoch sind ihre organoleptischen Eigenschaften noch nie beschrieben oder nahegelegt worden.

Somit betrifft die Erfindung auch die neuen I', deren Herstellung, Riechstoffkompositionen, die Verbindungen I bzw. I' als organoleptische Wirkstoffe enthalten, sowie die Verwendung der Verbindungen I als Riechstoffe.

Die Formel I umfasst auch die möglichen Isomeren, also z.B. im Falle I b) c) e) die cis/trans-Isomeren, im Falle I d) die Doppelbindungsisomeren, im Falle I f) die Diastereomeren.

Das erfindungsgemässe Verfahren zur Herstellung des erfindungsgemässen neuen I' ist dadurch gekennzeichnet, dass man
a) 3-tert.Butyl-phenol katalytisch unter Verwendung von Raney-Nickel als Katalysator zum Alkohol Ib hydriert und gegebenenfalls das so erhaltene 3-tert.Butyl-cyclohexanol entsprechend verestert, oder dass man
b) 3-tert.Butyl-cyclohexanon (Ia) zu einer Verbindung I mit X = d), f) oder g) derivatisiert.

Die Herstellung aller dieser Derivate geschieht durchwegs in an sich bekannter Weise, wobei die geeigneten Parameter die folgenden sind:

### Derivate I' b

α) Hydrierung eines Phenols mit Raney-Nickel in an sich bekannter Weise

| | |
|---|---|
| Temperaturbereich | ca. 100 bis ca. 170°C, vorzugsweise ca. 150°C |
| Druckbereich | ca. 10 bis ca. 50 bar, vorzugsweise ca. 20 bar (H₂) |
| Lösungsmittel | fakultativ, z.B. CH₃COOH, C₂H₅OH etc. |

β) Herstellung der Ester aus dem gemäss α) erhaltenen Alkohol
β1) Erhitzen des cyclischen Alkohols I' mit der entsprechenden Säure auf Rückflusstemperatur von Benzol, Toluol, etc., zweckmässigerweise am Wasserabscheider; Anwesenheit eines sauren Katalysators, wie einer Mineralsäure oder einer organischen Säure ist zwingend, z.B. Schwefelsäure, Phosphorsäure, p-Toluolsulfonsäure, etc .
β2) Analoge Umsetzung des Alkohols I' in demselben Lösungsmittel wie β1) mit einem Säurederivat anstelle der Säure, wie Anhydrid, oder Halogenid in Anwesenheit einer Base, z.B. einer organischen Base wie eines sekundären oder tertiären Amins, z.B. Pyridin, Piperidin, etc.

### Derivate Ic

Die Herstellung der Oxime geschieht zweckmässigerweise völlig konventionell ausgehend aus Ia, also z.B. mittels einem Hydroxylaminhydrohalogenid, z.B. dem -hydrochlorid, zweckmässigerweise wässrig, oder in Methanol, Aethanol, etc. und in Anwesenheit einer Base wie Pyridin, Natriumhydroxid, Na₂CO₃, etc.

### Derivate I' d)1 [T ist CN]

Knoevenagelkondensation von 3-tert.Butylcyclohexanon mit Cyanessigsäure (a), oder mit Acetonitril (b)
(a) Katalysator ist zweckmässigerweise eine schwächere Base wie NH₄•O₂CCH₃, Pyridin, Piperidin, Triäthylamin, etc.

| | |
|---|---|
| Lösungsmittel | z.B. Methanol, Benzol, Toluol, etc. |
| Reaktionstemperatur | vorzugsweise erhöht, insbes. Rückfluss- |
| | temperatur des Lösungsmittels. |

(b) in diesem Fall wird zweckmässigerweise in Anwesenheit einer stärkeren Base, beispielsweise eines Alkalihydroxids, z.B. KOH oder NaOH gearbeitet.

### Derivate I'd)2 [T ist CHO]

α) Behandlung von 3-tert.Butyloxycyclohexanon mit einem Acetylid (z.B. dem Li-, Na- oder K-acetylid, etc.) in DMF, einem anderen polaren Lösungsmittel oder auch in Ammoniak, etc.
β) Umlagerung des bekannten acetylenischen Alkohols Ie mittels Katalysatoren auf der Basis von Silylvanadaten. Ein bevorzugter Katalysator ist das tris(Triphenylsilyl)vanadat. Man arbeitet zweckmässigerweise in Lösungsmitteln wie Benzol, Toluol oder Xylol, etc. und zweckmässigerweise bei erhöhten Temperaturen, beispielsweise bei den Rückflusstemperaturen der Lösungsmittel.

Die Derivate f) und g) sind durch übliche Ketalisierung von 3-tert.Butylcyclohexanon mittels 1,4-Butandiol oder 1,2-Propandiol zugänglich. Die Ketalisierung wird nach an sich bekannter Weise, also katalytisch, zweckmässigerweise unter Verwendung einer starken Säure wie p-Toluolsulfonsäure und in Lösungsmitteln wie Benzol oder Toluol durchgeführt. Man arbeitet zweckmässigerweise bei erhöhten Temperaturen, und unter Verwendung eines Wasserabscheiders.

Die Reinigung der Verbindungen I' kann in an sich bekannter Weise erfolgen, z.B. durch Umkristallisation, Destillieren oder mittels Chromatographie.

Aufgrund ihrer wertvollen olfaktorischen Eigenschaften mit sehr breitem Spektrum eignen sich die Verbindungen I als Riechstoffe, und zwar insbesondere in Kombination mit der heutzutage zur Verfügung stehenden umfangreichen Palette von natürlichen und synthetischen Riechstoffe zur Kreation von Parfüm-Kompositionen, welche ihre Anwendung in allen Applikations-Segmenten finden können. Beispiele der zahlreichen bekannten Riechstoffingredientien natürlichen oder synthetischen Ursprungs, wobei die Palette der natürlichen Rohstoffe sowohl leicht- als auch mittel- und schwerflüchtige Komponenten, und diejenige der Synthetika Vertreter aus etlichen Stoffklassen umfassen kann, sind:
- Naturprodukte, wie Baummoos-Absolue, Basilikumöl, Agrumenöle (wie Bergamotteöl, Mandarinenöl, usw.), Mastix-Absolue, Myrtenöl, Palmarosaöl, Patchouliöl, Petitgrainöl, Wermutöl, Lavendelöl, Rosenöl, Jasminöl, Ylang-Ylangöl, Sandelholzöl,
- Alkohole, wie Farnesol, Geraniol, Linalool, Nerol, Phenyläthylalkohol, Rhodinol, Zimtalkohol, cis-3-Hexenol, Menthol, α-Tocopherol,
- Aldehyde, wie Citral, α-Hexylzimtaldehyd, Hydroxycitronellal, Lilial® (p-tert.Butyl-α-methyl-dihydrozimtaldehyd), Methylnonylacetaldehyd, Phenylacetaldehyd, Anisaldehyd, Vanillin,
- Ketone, wie Allyljonon, α-Jonon, β-Jonon, Isoraldein (Isomethyl-α-jonon), Verbenone, Nootkaton, Geranylaceton,
- Ester, wie Allyl-phenoxyacetat, Benzyl-salicylat, Cinnamylpropionat, Citronellyl-acetat, Decylacetat, Dimethylbenzylcarbinyl-acetat, Aethylacetoacetat, Aethyl-acetylacetat, cis-3-Hexenyl-isobutyrat, Linalylacetat, Methyl-dihydrojasmonat, Styrallylacetat, Vetiverylacetat, Benzylacetat, cis-3-Hexenylsalicylat, Geranylacetat, usw.
- Lactone, wie γ-Undecalacton, δ-Decalacton, Pentadecan-15-olid,
- verschiedene, in der Parfümerie oft benützte Komponenten, wie Indol, p-Menthan-8-thiol-3-on, Methyleugenol, Eugenol, Anethol.

Die unter Verwendung von Verbindungen I hergestellten Riechstoffkompositionen, insbesondere solcher mit Richtung blumig, aldehydisch, agreste, marin, ambrig, holzig, bestechen besonders durch ihre Originalität.

Bei ihrer Verwendung als Riechstoffe lassen sich die Verbindungen der Formel I (bzw. deren Gemische) in weiten Grenzen einsetzen, die beispielsweise von ca. 0,1 (Detergentien) bis ca. 30 Gewichtsprozent (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauchen kann. Die bevorzugten Konzentrationen bewegen sich zwischen ca. 0,5 und ca. 10 Gewichtsprozent. Die mit den Verbindungen I hergestellten Kompositionen lassen sich für alle Arten von parfümierten Verbrauchsgütern einsetzen (Eaux de Cologne, Eaux de Toilette, Extraits, Lotionen, Cremes, Shampoos, Seifen, Salben, Puder, Zahnpasten, Mundwässer, Desodorantien, Detergentien, Gewebeveredler, Tabak, usw.).

Die Verbindungen I können demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben aufgeführten bekannten Riechstoffe bzw. Riechstoffgemische nach (dem Parfümeur) bekannter Art und Weise verwendet werden, wie dies z.B. aus W.A. Poucher, Perfumes, Cosmetics and Soaps 2, 7. Auflage, Chapman und Hall, London, 1974 hervorgeht.

Dank ihrer hochstehenden olfaktorischen Eigenschaften werden die Verbindungen der Formel I bevorzugt in der Luxusparfümerie und in Kompositionen für Kosmetika zum Einsatz kommen. Bevorzugte Verbindungen sind das Acetat, das Keton und die Nitrile, siehe die Formel Ia, b und d.

Die nachstehenden Beispiele illustrieren die Erfindung.

In diesen folgenden Beispielen wurde das cis/trans-Verhältnis jeweils gaschromatographisch über das Flächenverhältnis ermittelt.

Dasselbe Prozedere diente zur Bestimmung der Doppelbindungsisomere (Beispiele 7,8) und der Diastereomere (Beispiel 9).

### Beispiel 1

300 g (2 Mol) 3-tert.Butylphenol werden in einem Autoklav vorgelegt. 30 g Raney-Nickel werden zugegeben und es wird nun bei 20 bar Wasserstoff und 160°C während 4 3/4 Stunden unter Rühren hydriert. Man lässt das Reaktionsgemisch auf Raumtemperatur abkühlen, verdünnt es mit wenig Aether, filtriert über Celite und dampft ein. Man erhält auf diese Weise 299,9 g (96%) eines Gemisches von trans-3-tert.Butylcyclohexanol (23%) und cis-3-tert.Butylcyclohexanol (77%).
Geruch des Gemischs: Erdig, moosig, holzig, camphrig, grün-würzig.
Spektrale Daten:
a) trans-3-tert.Butylcyclohexanol
   H-NMR (CDCl₃, 200 MHz): 0,85, s, 9H, t-Butyl; 4,14-4,215, breites Multiplett, 1H, -CHOH);
   MS: 138(5); 123(16); 99(15); 83(25); 82(38); 81(40), 80(21); 67(33); 57(100); 56(36); 55(25); 41(45); 29(23);
b) cis-3-tert.Butylcyclohexanol
   H-NMR (CDCl₃, 200 MHz): 0,86, s, 9H, t-Butyl; 3,47-3,63, breites Multiplett, 1H, CH-OH;
   MS: 123(13); 99(22); 83(25); 82(56); 81(33); 67(45); 57(100); 56(31); 55(28); 41(50); 29(25).

### Beispiel 2

Ein Gemisch von 109,39 g (0,7 Mol) 3-tert.Butylcyclohexanol (hergestellt gemäss Beispiel 1), 46,24 g (0,77 Mol) Essigsäure und 231 mg p-Toluolsulfonsäure, gelöst in 210 ml Toluol, wird am Wasserabscheider während 6 Stunden rückflussiert. Dann wird mit Wasser gewaschen, mit Aether extrahiert, mit einer 10% Bicarbonatlösung und Wasser gewaschen, getrocknet und eingedampft. Man erhält auf diese Weise 142 g Rohprodukt. Nach Destillation erhält man 105 g (76%) geruchlich einwandfreies Produkt, das 19% trans-3-tert.Butylcyclohexylacetat und 78% cis-3-tert.Butylcyclohexylacetat enthält.
Geruch des Gemischs: holzig, irisartig, frisch, blumig, leicht camphrig.
Spektrale Daten:
a) trans-3-tert.Butylcyclohexylacetat
   H-NMR (CDCl₃, 200 MHz): 0,84, s, 9H, t-Butyl; 2,06, s, 3H, CH₃-CO-O; 5,10-5,18, Multiplett CH-O-;
   MS: 141(17); 123(16); 83(15); 82(49); 81(40); 67(42); 61(14); 57(100); 43(39); 41(21);
b) cis-3-tert.Butylcyclohexylacetat
   H-NMR (CDCl₃, 200 MHz): 0,86, s, 9H, t-Butyl; 2,03, s, 3H, CH₃-CO-O; 4,60-4,78, breites Multiplett CH-O;
   MS: 142(2); 138(5); 123(21); 117(4); 95(3); 83(31); 82(84); 81(25); 80(35); 67(59); 61(27); 57(100); 43(48); 41(21); 29(5).

### Beispiel 3

A) 15,63 g (0,1 Mol) 3-tert.Butylcyclohexanol (hergestellt gemäss Beispiel 1), 8,7 g (0,11 Mol) Pyridin werden in 30 ml Toluol gelöst und 10,18 g (0,11 Mol) Propionylchlorid werden zugetropft. Dann wird auf 50°C erhitzt, während 4 Stunden gerührt, auf Wasser gegossen, mit Aether extrahiert, mit 2N Salzsäure, Wasser, 10% Natriumbicarbonatlösung und Wasser gewaschen, über Magnesiumsulfat getrocknet und eingedampft. Das Rohprodukt (21,76 g) wird zunächst einer ersten Destillation unterworfen. Das erhaltene Produkt (17,6 g) wird chromatographiert (Kieselgel 60 Merck, 0,04-0,063 mm 150 g 2% Aether in Hexan) und hierauf nochmals destilliert. Sdp.: 60°C/1 mbar. Man erhält auf diese Weise 16,04 g (75%) eines Gemisches von trans-3-tert.Butylcyclohexanolpropionat (21%) und cis-3-tert.Butylcyclohexanolpropionat(78%).
Geruch des Gemisches: fruchtig, nach Pampelmuse, aldehydig.
Spektrale Daten:
a) trans-3-tert.Butylcyclohexanolpropionat
      H-NMR (CDCl₃, 200 MHz): 0,83, s, 9H, t-Butyl; 5,12-5,19, Multiplett CH-O-;
      MS: 155(23); 138(5); 123(19); 95(3); 83(9); 82(42); 81(36); 80(29); 75(26);
      67(27); 57(100); 41(11); 29(9);
   b) cis-3-tert.Butylcyclohexanolpropionat
      H-NMR (CDCl₃, 200 MHz): 0,86, s, 9H, t-Butyl; 4,62-4,78, breites Multiplett CH-O;
      MS: 138(7); 131(3); 123(16); 83(14); 82(58); 81(17); 80(29); 75(44); 67(42); 57(100); 41(16); 29(14).

B) Analog dem oben unter A) beschriebenen Verfahren wird auch 3-tert.Butylcyclohexylacrylat durch Behandlung von 3-tert.Butylcyclohexanol mit Acrylsäurechlorid in Toluol in Gegenwart von Pyridin hergestellt.
   Geruch: frisch, grün, fruchtig, Tomate, Pflaume.
   Spektrale Daten
   des Gemisches cis/trans 3-tert.Butylcyclohexylacrylat im Verhältnis 76/23.
   IR: 1725, 1618, 1630 cm⁻¹
   MS: 153(4); 138(4); 123(14); 82(58); 73(26); 67(36); 57(100); 55(52); 41(24); 27(13).

### Beispiel 4

Ein Gemisch von 15,63 g (0,1 Mol) 3-tert.Butylcyclohexanol (hergestellt gemäss Beispiel 1), 9,70 g (0,11 Mol) Isobuttersäure und 100 mg p-Toluolsulfonsäure, gelöst in 100 ml Toluol, wird am Wasserabscheider während 5 Stunden und 40 Minuten rückflussiert. Dann wird mit Wasser gewaschen, mit Aether extrahiert, mit gesättigter Bicarbonatlösung und Wasser gewaschen, getrocknet und eingedampft. Man erhält auf diese Weise 22 g Rohprodukt. Dieses Produkt wird chromatographiert (Kieselgel 60 Merck 150 g, 0,04-0,063 mm 180 g, Eluierungsmittel 2% Aether in Hexan) und hierauf destilliert. Man erhält auf diese Weise 13,2 g (58%) eines Gemisches von trans-3-tert.Butylcyclohexanolisobutyrat (22%) und cis-3-tert.Butylcyclohexanolisobutyrat (77%).

Geruch des Gemisches: fruchtig (Aprikose), holzig, lactonartig, grün, aldehydrig, blumig.
Spektrale Daten:
a) trans-3-tert.Butylcyclohexanolisobutyrat
   H-NMR (CDCl₃, 200 MHz): 0,83, s, 9H, t-Butyl; 1,18, dd, J=7,J=0,5; 2,43-2,64, Multiplett, J=7Hz, CH(CH₃)₂; 5,10-5,1, Multiplet CH-O-;
   MS: 169(5); 138(5); 123(8); 89(33); 83(11); 82(26); 81(22); 80(23); 71(16); 67(19); 57(100); 56(7); 5(9); 43(26); 41(20);
b) cis-3-tert.Butylcyclohexanolisobutyrat
   H-NMR (CDCl₃, 200 MHz): 0,86, s, 9H, t-Butyl; 1,16, d, J=7Hz; 2,38-2,60, Multiplett, J=7Hz, CH(CH₃)₂; 4,60-4,76, breites Multiplet CH-O;
   MS: 145(2); 138(6); 123(11); 89(43); 83(15); 82(36); 81(14); 80(22); 71(15); 69(7); 67(24); 57(100); 43(28); 41(17).

### Beispiel 5

15,63 g (0,1 Mol) 3-tert.Butylcyclohexanol (hergestellt gemäss Beispiel 1), 8,7 g (0,11 Mol) Pyridin werden in 30 ml Toluol gelöst und 10,4 g (0,11 Mol) Chlorameisensäuremethylester wird zugetropft. Man beobachtet nun leichte Exothermie und Bildung eines Niederschlages. Es wird unter Rühren 5 Stunden auf 50°C erhitzt und noch einmal ein Aequivalent Pyridin (7,9 g) und ein Aequivalent Chlorameisensäure (9,45 g) zugegeben. Nach 22 1/2 Stunden wird mit Wasser versetzt, mit Aether extrahiert, mit 2N Salzsäure, Wasser, gesättigter Bicarbonatlösung und Wasser gewaschen, über Magnesiumsulfat getrocknet und eingedampft. Das Rohprodukt (15,84 g) wird chromatographiert (Kieselgel 60 Merck, 0,04-0,063 mm) 150 g Eluierungsmittel (2% Aether in Hexan) und destilliert. Man erhält auf diese Weise 7,96 g (37%) eines Gemisches von trans-3-tert.Butylcyclohexylmethylcarbonat (9%) und cis-3-tert.Butylcyclohexylmethylcarbonat (81%).
Geruch des Gemisches: grün, holzig, blumig, fruchtig, würzig, erdig.
Spektrale Daten:
a) trans-3-tert.Butylcyclohexylmethylcarbonat
   H-NMR (CDCl₃, 200 MHz): 0,84, s, 9H, t-Butyl; 3,78, s, CH₃-O; 4,98-5,07, Multiplett CH-O-;
   MS: 157(14); 138(3); 123(17); 83(21); 82(50); 81(42); 80(21); 77(47); 67(47); 57(100); 41(27);
b) cis-3-tert.Butylcyclohexylmethylcarbonat
   H-NMR (CDCl₃, 200 MHz): 0,86, s, 9H, t-Butyl; 3,77, s, CH₃-.O; 4,46-4,64, Multiplett CH-O;
   MS: 159(15); 133(3); 123(24); 83(27); 82(90); 81(24); 80(13); 77(85); 67(57); 57(100); 56(21); 55(14); 41(25).

### Beispiel 6

300 g (2 Mol) 3-tert.Butylphenol werden in Gegenwart von 3 g Palladium auf Aktivkohle 5% und 3 g wasserfreiem Natriumcarbonat bei 0,15-0,3 bar Wasserstoffüberdruck und 115°C in einem Autoklav während 21 Stunden hydriert. Dann wird über Celite filtriert. Das Celite wird mit Aether gewaschen und das Gelöste eingedampft. Auf diese Weise erhält man 310,8 g Rohprodukt, nämlich 1,5% trans-tert.Butylcyclohexanol, 3,5% cis-tert.Butylcyclohexanol, 86% 3-tert.Butylcyclohexanon und 7,6% 3-tert.Butylphenol. Dieses Produkt kann durch Destillation gereinigt werden. Man erhält 232 g (75%) 3-tert.Butylcyclohexanon, Sdp. 75-76°C, 2 mbar (geruchlich akzeptabel, Reinheit 94%) neben 11,3 g (3,6%) leicht verunreinigtes Material (Reinheit 92%).
Geruch: camphrig, aromatisch, grün, erdig, holzig, pinienartig, fruchtig, minzig.
Spektrale Daten
3-tert.Butylcyclohexanon
H-NMR (CDCl₃, 200 HHz): 0,90, s, tert.Butyl;
MS: 154(16); 139(3); 99(14); 98(81); 97(24); 83(24); 70(18); 69(29); 57(100); 55(33); 41(54); 29(14).

### Beispiel 7

8,42 g (0,15 Mol) KOH werden zu 24,63 g Acetonitril zugegeben, und es wird unter Rühren auf Rückflusstemperatur erhitzt. Es wird eine Lösung von 15,43 g (0,1 Mol) 3-tert.Butylcyclohexanon in 6,16 g Acetonitril zugetropft. Es wird 2,5 Stunden am Rückfluss unter Rühren erhitzt, dann auf Wasser gegossen, mit Aether extrahiert, mit Wasser gewaschen, über Magnesiumsulfat getrocknet und eingedampft. Das Rohprodukt (18,4 g) wird chromatographiert (Kieselgel 60 Merck, 0,04-0,063 mm, 150 g, Elution: mit 2% Aether in Hexan) und destilliert. Man erhält auf diese Weise 6,39 g (36%) Rohprodukt. Dieses Produkt enthält 4 Isomere im Verhältnis 9/40/9/40.

Gemäss NMR-Spektrum bestehen die zwei Hauptisomere aus E- und Z-3-tert.Butylcyclohexylidenacetonitril d)12 und d)14 und die Nebenkomponente aus (3-tert.Butyl-cyclohex-1-enyl)-acetonitril und (3-tert.Butyl-cyclohex-6-enyl)-acetonitril d)11 und d)13.

Geruch des Gemisches: würzig (kumin), grün, erdig, aldehydisch, nach Anis, aromatisch, lederartig.
Spektrale Daten
H-NMR (CDCl₃, 200 MHz): Gemisch d)1
0,880, s, tert.Butyl d)12 oder d)14;
0,92, s, tert.Butyl d)12 oder d)14;
0,881, s, tert.Butyl d)11 oder d)13;
0,882, s, tert.Butyl d)11 oder d)13;
5,03-5,07, Multiplett, 1H, d)12 und d)14, C=CH-CN;
5,75-5,82, breites Singulett d)11 und d)13, CH=C-CH₂-CN;

MS d)11:
   177(6); 162(3); 121(16); 106(4); 94(56); 79(29); 57(100); 41(30); 32(12); 28(36);
MS d)12:
   177(17); 163(13); 162(85); 122(24); 121(83); 120(63); 106(18); 97(30); 96(12); 94(44); 93(48); 91(12); 81(21); 80(33); 79(29); 77(18); 57(100); 56(12); 55(47); 43(21); 41(75);
MS d)13:
   177(6); 162(2); 121(17); 106(8); 93(7); 81(16); 80(13); 79(16); 69(9); 57(1oo); 41(28); 28(2); 93(19); 84(10); 81(20); 80(15); 79(16); 77(10); 69(16); 57(100); 55(39); 43(16); 41(69); 39(15); 29(15).
MS d)14:
   177(22); 162(48); 134(7); 122(12); 121(34); 120(27); 106(12); 97(37); 96(12); 94(18);

### Beispiel 8

Ein Gemisch von 15,43 g (0,1 Mol) 3-tert.Butylcyclohexanon, 0,4 g Ammoniumacetat und 50 ml Toluol wird am Wasserabscheider auf Rückflusstemperatur erhitzt, dann werden 7,32 g (86 mM) Cyanessigsäure und 1,2 g (20 mM) Essigsäure in kleinen Portionen zugegeben. Nach 23 Stunden Rückflusstemperatur wird abgekühlt, mit Wasser gewaschen, mit Aether verdünnt, mit gesättigter Natriumbicarbonatlösung, dann mit Wasser gewaschen und eingedampft. Das Rohprodukt (19,52 g) wird destilliert. Auf diese Weise erhält man 10 g (56% Ausbeute) bestehend hauptsächlich aus den zwei Hauptisomeren d)11 und d)13 und Spuren der Verbindungen d)12 und d)14
(d)11: 57,3%, d)12: 1%, d)13: 39,3%, d)14: 1,7%).

### Beispiel 9

15,43 g (0,1 Mol) 3-tert.Butylcyclohexanon, 8,37 g (0,11 Mol) 1,2-Propandiol und 100 mg p-Toluolsulfonsäure wird in 100 ml Toluol gelöst und am Wasserabscheider rückflussiert. Nach 5 Stunden Rückfluss wird abgekühlt und mit Wasser gewaschen, mit Aether extrahiert. Die organische Phase wird mit einer Bicarbonatlösung und Wasser gewaschen. Auf diese Weise erhält man 21,93 g eines Rohproduktes, das hauptsächlich aus drei Isomeren besteht (Verhältnis der Isomere ca. 2/1/1). Der vierte mögliche Isomer wird wahrscheinlich unter den gaschromatographischen Bedingungen nicht aufgetrennt.
Geruch: fruchtig, grün, holzig, blumig, anisartig, aromatisch, camphrig, tabakartig.
Spektrale Daten:
2-Methyl-7-tert.butyl-1,4-dioxaspiro[4,5]decan
H-NMR (Gemisch der zwei Isomere im Verhältnis 2/1; CDCl₃, 200 MHz): 0,84, s, 9H, tert.Butyl; 1,26, d, J=6Hz, 3H, CH₃-CH-; 1,28, d, J=6Hz, 3H, CH₃-CH-; 3,38-3,50, Multiplett, 1H; 3,9-4,10, Multiplett, 1H; 4,11-4,32, Multiplett, 1H;
MS f)1: 156(11); 155(100); 114(13); 113(91); 111(9); 100(4); 97(28); 69(15); 57(10); 55(35); 41(16);
MS f)2: 156(12); 155(100); 114(8); 113(66); 111(9); 97(33); 79(2); 69(15); 55(33); 41(20);
MS f)3: 155(80); 113(100); 111(14); 97(39); 69(33); 57(33); 56(10); 55(93); 43(21); 42(16); 41(69); 39(11); 29(18); 27(11).

### Beispiel 10

15,43 g (0,1 Mol) 3-tert.Butylcyclohexanon, 9,91 g (0,11 Mol) 1,4-Butandiol und 0,1 g p-Toluolsulfonsäure werden in 100 ml Toluol am Wasserabscheider rückflussiert. Nach 18 Stunden wird abgekühlt, mit Wasser gewaschen und mit Aether extrahiert. Die organische Phase wird mit 10% Dicarbonatlösung, dann mit Wasser gewaschen, über Magnesiumsulfat getrocknet und eingedampft. Das Rohprodukt (19,2 g) wird chromatographiert (Kieselgel Merck 60, 0,04-0,063 mm 150 g, Elution mit 1% Aether in Hexan) und destilliert (Sdp. 108°C/1 mbar). Man erhält auf diese Weise 8,11 g (36%) 9-tert.Butyl-1,6-dioxaspiro[5,6]dodecan.
Geruch: holzig, blumig, aromatisch, campherig, fruchtig, minzig, erdig.
Spektrale Daten
H-NMR (CDCl₃, 200 MHz): 0,86, s, 9H, tert.Butyl; 3,58-3,78, breites Doublett, 4H, J=14Hz;
MS: 226(8); 170(23); 169(90); 139(35); 128(15); 127(79); 115(18); 98(22); 97(69); 73(12); 69(26); 57(24); 55(100); 43(19); 41(46); 28(24).

### Beispiel 11

Zu 8,11 g (45 mM) 3-tert.Butyl-1-ethyn-cyclohexan-1-ol (cis,trans-Gemisch) gelöst in 80 ml Xylol, werden 2,2 g Triphenylsilanol, 0,16 g Stearinsäure und 0,12 ml Vanadiumisopropylat zugegeben, und das Gemisch wird 10 Stunden auf Rückflusstemperatur erhitzt. Man lässt 12 Stunden stehen. Es wird mit 50 ml gesättigter Natriumbicarbonat-lösung gewaschen, mit Aether extrahiert, mit Wasser gewaschen, getrocknet und eingedampft. Das Rohprodukt (13,4 g) enthält
10% 3-tert.Butyl-1-acetaldehyd-1-cyclohexen d)21 oder d)22,
10% 3-tert.Butyl-1-acetaldehyd-6-cyclohexen d)21 oder d)22,
21,4% E- oder Z-3-tert.Butylcyclohexylidenacetaldehyd d)23 oder d)24,
24,2% E- oder Z-3-tert.Butylcyclohexylidenacetaldehyd d)23 oder d)24.
Nach Destillieren erhält man die Fraktionen D1, D2 und D3:
D1: 1 g 42% d)21, 27% d)22
D2/D3: 4,34 g 28% d)21, 37% d)22, 20% d)23, 8% d)24.
Sp: 48-52°/0,045 Torr.
Anscheinend hat sich das Produkt während der Destillation isomerisiert.
Spektrale Daten
3-tert.Butyl-1-acetaldehyd-1-cyclohexen d)21 oder d)22
   3-tert.Butyl-1-acetaldehyd-6-cyclohexen d)21 oder d)22
H-NMR (CDCl₃, 200 MHz): Gemisch d)21 + d)22
   0,87, 0,88, 9H, tert.Butyl; 2,97-3,04, 2H, breites Singlett, CH₂-CO; 5,57-5,65, 1H, breites Singlett, CH=C; 9,59 (dd, J=3Hz, J=3Hz), 1H, OCH-; 9,61 (dd, J=3Hz, J=3Hz), 1H, OCH-;
MS d)21:
   180(1); 124(48); 95(19); 93(10); 81(15); 80(49); 79(34); 67(11); 57(100); 41(35); 29(14);
MS d)22:
   180(1); 124(46); 95(19); 93(7); 81(16); 80(39); 79(23); 67(11); 57(100); 41(32); 39(10); 29(13).
E-3-tert.Butylcyclohexylidenacetaldehyd d)23 oder d)24
   Z-3-tert.Butylcyclohexylidenacetaldehyd d)23 oder d)24
H-NMR (CDCl₃, 200 MHz): d)23 oder d)24
   0,90. s. 9H, tert.Butyl; 3,3-3,42, breites Doublett, J=13, 1H; 5,84, d, J=9, CH=C; 10,0, d, J=9Hz, OCH;
H-NMR (CDCl₃, 200 MHz): d)23 oder d)24
   0,92, s, 9H, tert.Butyl; 3,4-3,52 breites Doublett, J=13, 1H; 5,84, d, J=9, CH=C; 10,04, d J=9, OCH;
MS d)23:
   180(0,2); 165(2); 123(100); 105(8); 95(17); 81(16); 80(11); 79(15); 67(16); 57(33); 55(14); 43(14); 41(28); 39(9); 29(13);
MS d)24:
   180(34); 163(4); 137(7); 124(16); 123(65); 109(23); 96(17); 95(37); 93(13); 91(11); 81(32); 80(30); 79(27); 77(13); 67(27); 57(100); 55(35); 53(14); 43(25); 41(55); 39(18); 29(24).

### Beispiel 12

### Riechstoffkompositionen

In den Beispielen a) bis n) bezieht sich die jeweils an erster Stelle und in Klammer gesetzte Komponente auf das Reaktionsprodukt des entsprechenden Synthesebeispiels.

### a) Parfumbase, für Seife geeignet, Typ blumig

| | Gewichtsteile |
|---|---|
| (2) | 30,00 |
| Benzylacetat | 150,00 |
| Linalylacetat | 40,00 |
| Phenyläthylalkohol | 50,00 |
| n-Decylaldehyd | 4,00 |
| Undecylenaldehyd | 5,00 |
| Laurinaldehyd | 5,00 |
| Ciste Absolue | 1,00 |
| Cumarin | 70,00 |
| Dipropylenglykol | 261,00 |
| Estragol (n-Allylanisol) | 1,00 |
| Aethyl-vanillin 10%/DIP | 5,00 |
| Geraniol intermediate 60 (3,7-Dimethyl-octa-2,6-dienol) | 10,00 |
| Girofle Feuilles Ess. (Gewürznelkenessenz) | 15,00 |
| Indolen (8,8-Di-(1H-indol-1-yl)-2,6-dimethyl-octan-2-ol) | 1,00 |
| Ionanthem 100% (α-Jonon) | 20,00 |
| Isoeugenol | 10,00 |
| Isoraldein 70 (Methyljonone) | 65,00 |
| Lilial | 30,00 |
| Linalool | 45,00 |
| Xylol-moschus | 25,00 |
| Oranger krist. [1-(2-Naphthanenyl)äthanon] | 2,00 |
| Petitgrain Ess. Paraguay | 5,00 |
| Styrax | 15,00 |
| α-Terpinol | 30,00 |
| Vetiver Ess. Java | 25,00 |
| Ylang Ylang Ess. | 80,00 |
| | Total: 1000,00 |

In obiger Komposition verstärkt die Verbindung I die holzigen und Iris-Noten, und erbringt Stärke der blumigen Komposition.

### b) Eau de Toilette Masculine, Typ holzig bis ambrig

| | Gewichtsteil |
|---|---|
| (2) | 30,00 |
| Ambrettolid | 2,00 |
| Ambroxan [3aR-(3aα,5aβ,9aα,9bβ)-Dodecahydro-3a,6,6a,9a-tetramethyl-naphtho[2,1-B]furan] | 2,00 |
| Bergamotte Ess. | 200,00 |
| Cedrylmethyläther | 80,00 |
| Citronen Ess. | 60,00 |
| Citronellol extra | 40,00 |
| Civette absolue | 1,00 |
| Cumarin | 15,00 |
| Cyclohexal | 70,00 |
| Dipropylenglykol | 20,00 |
| Hedion | 200,00 |
| Isoraldein 70 | 50,00 |
| Lavandin | 60,00 |
| Methylcedrylketon | 50,00 |
| Patchouli Ess. | 100,00 |
| Sandalore | 15,00 |
| Vanillin | 5,00 |
| | Total: 1000,00 |

In dieser Komposition erbringt die Verbindung I (von holzigem und ambrigem Charakter) Harmonie, sie akzentuiert den Frischeaspeklt und den Impakt.

### c) Parfümbase für Shampoos, vom blumigen, aldehydischen Typ

| | Gewichtsteile |
|---|---|
| (2) | 40,00 |
| Hexylzimtaldehyd | 350,00 |
| Laurinaldehyd 10%/DIP | 5,00 |
| Aldehyd iso C₁₁ (10%/DIP) (Gemisch 10-Undecenal und cis,trans 9-Undecenal) | 5,00 |
| Citronellol | 60,00 |
| Cumarin | 35,00 |
| β-Dihydro-jonon | 10,00 |
| Dipropylenglykol | 40,00 |
| Ebanol (3-Methyl-5-(2,2,3-trimethyl-3-cyclopent-1-yl)-4-penten-2-ol) | 1,00 |
| Fixolid | 100,00 |
| Gardenol (Methylphenylcarbinylacetat) | 7,00 |
| Geraniol intermediate 60 | 60,00 |
| Givescone | 2,00 |
| Heliotropin | 20,00 |
| Iso E super | 25,00 |
| Isoraldein 40 | 70,00 |
| Lilial | 100,00 |
| Methyl-cedryl-keton | 40,00 |
| Ylang Ylang Ess. | 30,00 |
| | Total: 1000,00 |

In obiger Komposition verbindet die Verbindung I die blumigen, grünen, aldehydischen Kopfnoten mit den holzigen Noten, und verleiht der Komposition das nötige Volumen.

### d) Mehrzweckbase für ide Parfumerie Typ agreste, Meer, Chypre

| | Gewichtsteile |
|---|---|
| (8) | 30,00 |
| Methylnonylaldehyd | 10,00 |
| Aldehyde Mandarin (Dodec-2-en-1-al) | 10,00 |
| Armoise Ess. | 150,00 |
| Calone (7-Methyl-3,4-dihydro-2H-1,5-benzodioxepin-3-on) | 5,00 |
| Cashmeran (1,2,3,5,6,7-Hexahydro-1,1,2,3,3-penta methyl-4H-inden-4-on) | 5,00 |
| Clonal (Dodecanitril) | 30,00 |
| Cumarin | 5,00 |
| Dipropylenglykol | 370,00 |
| Evernyl (2,4-Dihydroxy-3,6-dimethyl-benzoesäure-methylester) | 20,00 |
| Florhydral (3-(3-Isopropylphenyl)-butanal) | 10,00 |
| Hedion | 55,00 |
| Homofuronol 20%/PG | 1,00 |
| Isobutyl-chinolin (2-(2-Methylpropyl)chinolin | 2,00 |
| Methyl-2-nonynoat | 1,00 |
| Rosenoxid 10% in Carbitol (Tetrahydro-4-methyl-2-(2-methyl-1-propenyl)-2H-pyran) | 10,00 |
| Benzylsalicylat | 170,00 |
| Tagetes Ess. | 1,00 |
| Thymianessenz | 5,00 |
| Tropional (α-Methyl-1,3-benzodioxole-5-propanal) | 100,00 |
| Undecatrien | 10,00 |
| | Total: 1000,00 |

Die Verbindung I liefert die typisch lederartige Tonalität und verstärkt den modernen Meerescharakter.

### e) Parfüm für Seife

| | Gewichtsteile |
|---|---|
| (7) | 5,00 |
| Benzylacetat | 40,00 |
| Dimethylbenzylcarbinolacetat | 10,00 |
| Phenyläthylalkohol | 120,00 |
| Hexylzimtaldehyd | 100,00 |
| Undecylenaldehyd | 3,00 |
| Butylhydroxytoluol | 2,00 |
| Citronen Ess. Reconstitution | 70,00 |
| Citronellol | 80,00 |
| Cumarin | 20,00 |
| γ-Decalacton | 3,00 |
| Dihydromyrcenol | 40,00 |
| Eugenol | 10,00 |
| Fixolid | 50,00 |
| Gardenol | 10,00 |
| Hedion | 50,00 |
| Isoraldein 70 | 80,00 |
| Lilial | 40,00 |
| Linalol | 30,00 |
| Methyl-cedryl-keton | 60,00 |
| Benzylsalicylat | 147,00 |
| Ylang Ylang Ess. | 30,00 |
| | Total: 1000,00 |

Die Komposition gewinnt an Impact und Frische durch die Anwesenheit von I, bedingt durch deren Stärke, grünem Wurzelgeruch und holzigem und Vetivercharakter.

### f) Eau de Toilette masculin, vom frischen, holzigen, ambrigen Typ

| | Gewichtsteile |
|---|---|
| (5) | 30,00 |
| Linalylacetat | 20,00 |
| Bergamotte Ess. | 100,00 |
| Sandelholzessenz | 30,00 |
| Cumarin | 3,00 |
| Cyclal C (3,5-Dimethyl-cyclohex-3-en-1-carboxaldehyd) | 1,00 |
| Cyclohexal (4-(4-Hydroxy-4-methylpentyl)cyclohex-3-en-1-carboxaldehyd) | 60,00 |
| Dihydro-myrcenol | 20,00 |
| Dipropylenglykol | 232,00 |
| Evernyl | 3,00 |
| Fixolid | 140,00 |
| Hedion | 20,00 |
| Indol 10%/DIP | 3,00 |
| β-Jonon | 5,00 |
| Iso E Super (1-(1,2,3,4,5,6,7,8-Octahydro-2,3,8,8-tetramethyl-2-naphthenyl)-äthanon) | 220,00 |
| Isobutylchinolin | 2,00 |
| Methyl-cedryl-keton | 100,00 |
| Methyloctincarbonat | 8,00 |
| Patchouli Ess. | 10,00 |
| Vanillin 10% DIP | 3,00 |
| | Total: 1000,00 |

Durch die Verbindung I gewinnt die Komposition an samtigem Charakter der holzigen und ambrigen Noten, der eindrückliche Accord ist grösstenteils Stärke und dem Volumen von I zu verdanken.

### g) Parfüm für Seife

| | Gewichtsteile |
|---|---|
| Oxim I'c) | 10,00 |
| Bornylacetat | 70,00 |
| Vetivenylacetat | 200,00 |
| Bergamotte Ess. | 100,00 |
| Citronen Ess. | 100,00 |
| Dihydromyrcenol | 50,00 |
| Dipropylenglykol | 205,00 |
| Ebanol | 2,00 |
| Eugenol | 10,00 |
| Evernyl | 3,00 |
| Fixolid | 40,00 |
| Isoraldein 40 | 30,00 |
| Lavandin Ess. | 100,00 |
| Pfefferminzöl | 10,00 |
| Romarin Ess. | 30,00 |
| Sauge Sclarée Ess. (Muskateller Salbeiöl) | 30,00 |
| Thymian Ess. | 10,00 |
| | Total: 1000,00 |

Die Verbindung I ist von ausgesprochenem aromatischem Charakter, holzig und camphrig, sie verleiht der Komposition Frische und Natürlichkeit, sie verstärkt die Noten vom Typ agreste.

### h) Parfumbase für Seife, Typ blumig, aldehydisch, holzig

| | Gewichtsteile |
|---|---|
| (1) | 5,00 |
| Vetivenyl-acetat | 30,00 |
| Decyl-acetat | 3,00 |
| Undecyl-aldehyd | 4,00 |
| Laurinaldehyd | 7,00 |
| Cumarin | 60,00 |
| Dipropylenglykol | 166,00 |
| Irisanthem (3-Methyl-4-(2,6,6-trimethyl-2-cyclohexen-1-yl)-3-buten-2-on) | 170,00 |
| Jasmin Reconstitution | 100,00 |
| Ketonmoschus | 70,00 |
| Phixia (Hydroxycitronellal) | 200,00 |
| Rosen Reconstitution | 80,00 |
| Vanillin | 5,00 |
| Ylang Ylang Ess. | 100,00 |
| | Total: 1000,00 |

Die Verbindung I weist eine kräftige grüne, camphrige Note auf. Sie verleiht der Komposition Frische und verstärkt den holzigen und vetiverartigen Charakter.

### i) Parfumbase für Seife, agreste

| | Gewichtsteile |
|---|---|
| (6) | 30,00 |
| Bornylacetat | 100,00 |
| Vetivenylacetat | 200,00 |
| Bergamotte Ess. | 100,00 |
| Citronen Ess. | 100,00 |
| Dihydromyrcenol | 50,00 |
| Dipropylenglykol | 171,00 |
| Ebanol | 1,00 |
| Eugenol | 10,00 |
| Evernyl | 3,00 |
| Isoraldein 40 | 30,00 |
| Lavandin Ess. | 100,00 |
| Pfefferminzöl | 10,00 |
| Ketonmoschus | 25,00 |
| Romarin Ess. | 30,00 |
| Sauge Sclarée Ess. | 30,00 |
| Thymian Ess. | 10,00 |
| | Total: 1000,00 |

Die Verbindung I erbringt, bedingt durch ihre frische, grüne Note und ihrer Thuyon- und Beifuss-Charakter die nötige Stärke, Kraft und Frische der Komposition.

### k) Parfumbase für Shampoos, Typ aldehydisch

| | Gewichtsteile |
|---|---|
| (9) | 10,00 |
| Zimtaldehyd | 350,00 |
| Laurinaldehyd 10%/DIP | 5,00 |
| Aldehyd iso C11 (10%/DIP) (Gemisch 10-Undecenal und cis,trans 9-Undecenal) | 5,00 |
| Citronellol | 60,00 |
| Cumarin | 35,00 |
| β-Dihydrojonon | 10,00 |
| Dipropylenglykol | 72,00 |
| Ebanol | 1,00 |
| Fixolid | 100,00 |
| Gardenol | 7,00 |
| Geraniol | 60,00 |
| Heliotropin | 20,00 |
| Iso E super | 25,00 |
| Isoraldein 40 | 70,00 |
| Lilial | 100,00 |
| Methyl-cedryl-keton | 40,00 |
| Ylang Ylang Ess. | 30,00 |
| | Total: 1000,00 |

Die Verbindung I weist einen holzigen, irisartigen und grünen Charakter auf, dadurch verbindet sie die holzigen, irisartigen milden aldehydisch blumigen Noten der Komposition, ferner verleiht sie der Komposition Volumen.

### l) Parfumbase für Shampoos, aldehydisch, kosmetisch

| | Gewichtsteile |
|---|---|
| (4) | 20,00 |
| Hexylzimtaldehyd | 350,00 |
| C₁₂-Aldehyd 10%/DIP | 5,00 |
| Aldehyd iso C11 (10%/DIP) (Gemisch 10-Undecenal und cis,trans 9-Undecenal) | 5,00 |
| Citronellol extra | 60,00 |
| Cumarin | 35,00 |
| Dipropylenglykol | 61,00 |
| Ebanol | 2,00 |
| Fixolid | 100,00 |
| Gardenol | 7,00 |
| Geraniol | 60,00 |
| Heliotropin | 20,00 |
| β-Jonon | 10,00 |
| Iso E super | 25,00 |
| Isoraldein 40 | 70,00 |
| Lilial | 100,00 |
| Methyl-cedryl-keton | 40,00 |
| Ylang Ylang Ess. | 30,00 |
| | |
| | Total: 1000,00 |

Die Komposition gewinnt an samtigem und vollem Charakter durch die Anwesenheit von I bzw. deren fruchtigen, lactonigen, holzigen Noten.

### m) Parfumbase für Shampoo, aldehydisch

| | Gewichtsteile |
|---|---|
| (3) | 80,00 |
| Hexylzimtaldehyd | 350,00 |
| C₁₂-Aldehyd 10%/DIP | 5,00 |
| Aldehyd iso C11 (10%/DIP) | 5,00 |
| Citronellol | 60,00 |
| Cumarin | 35,00 |
| β-Dihydrojonon | 10,00 |
| Ebanol | 1,00 |
| Fixolid | 100,00 |
| Gardenol | 7,00 |
| Geraniol intermediate 60 | 60,00 |
| Givescone | 2,00 |
| Heliotropin | 20,00 |
| Iso E super | 25,00 |
| Isoraldein 40 | 70,00 |
| Lilial | 100,00 |
| Methyl-cedryl-keton | 40,00 |
| Ylang Ylang Ess. | 30,00 |
| | Total: 1000,00 |

In diesem insbesondere für Shampoos geeigneten Parfum trägt die Verbindung I den nötigen Körper und das nötige Volumen bei, und zwar aufgrund ihrer holzigen und fruchtigen Note.

### n) Parfumbase für Seife; Note, blumig nach Orangen und holzig

| | Gewichtsteile |
|---|---|
| (11) | 5,00 |
| Benzylacetat | 150,00 |
| Linalylacetat | 40,00 |
| Phenyläthylalkohol | 50,00 |
| Ciste Abs. | 1,00 |
| Coumarin | 70,00 |
| Dipropylenglykol | 300,00 |
| Estragol | 1,00 |
| Aethylvanillin 10%/DIP | 5,00 |
| Geraniol Intermediate 60 | 10,00 |
| Girofle feuilles Ess. (Nelkenblätteröl) | 15,00 |
| Indolen | 1,00 |
| Ionanthem 100% | 20,00 |
| Isoeugenol | 10,00 |
| Isoraldein 70 | 65,00 |
| Lilial | 30,00 |
| Linalool | 45,00 |
| Xylolmoschus | 25,00 |
| Oranger krist. | 2,00 |
| Petitgrain Ess. | 5,00 |
| Styrax | 15,00 |
| α-Terpineol | 30,00 |
| Vetiver Ess. Java | 25,00 |
| Ylang Ylang-Oel | 80,00 |
| | Total: 1000,00 |

In dieser Parfumkomposition trägt die grüne und aldehydische Note von I ausgesprochen zum Impact bei; desgleichen bringt I viel grüne Frische in die Kopfnote der Komposition.

## Patentansprüche

1. Riechstoffkomposition, gekennzeichnet durch einen Gehalt an einer Verbindung der allgemeinen Formel 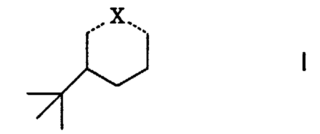 worin ...X... eine der Bedeutungen
a) >C=O
b) >CH-OR
worin R H, COCH₃, COCH₂CH₃, CO-CH(CH₃)₂, CO-CH=CH₂, COOCH₃ ist
wobei das Verhältnis cis/trans in den Isomeren von I 65-95:35-5 beträgt,
c) >C=N-OH
d) 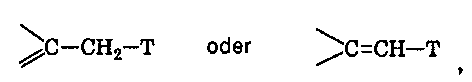 wobei T = CN oder CHO
e) 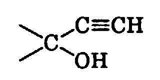
f) 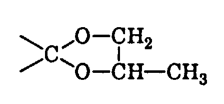 oder
g) 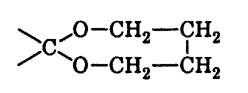 hat.

2. Verbindungen der Formel 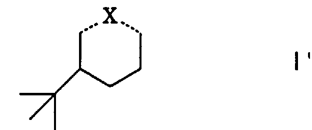 worin ...X... eine der Bedeutungen
b) >CH-OR
worin R H, COCH₃, COCH₂CH₃, CO-CH(CH₃)₂, CO-CH=CH₂, COOCH₃ ist
wobei das Verhältnis cis/trans in den Isomeren von I 65-95:35-5 beträgt,
d) 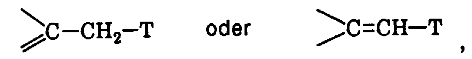 wobei T = CN oder CHO
f) 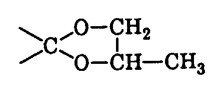 oder g) 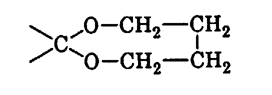
hat.

3. Verwendung einer Verbindung der Formel 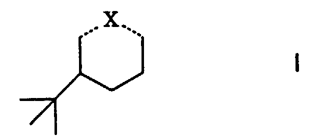 worin ...X... eine der Bedeutungen
a) >C=O
b) >CH-OR
worin R H, COCH₃, COCH₂CH₃, CO-CH(CH₃)₂, CO-CH=CH₂, COOCH₃ ist
wobei das Verhältnis cis/trans in den Isomeren von I 65-95:35-5 beträgt,
c) >C=N-OH
d) 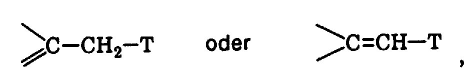 wobei T = CN oder CHO
e) 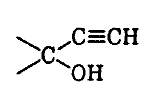
f) 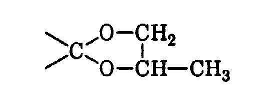
g) 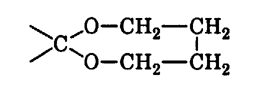 hat,
als Riechstoff.

4. Verfahren zur Herstellung der Verbindungen der Formel I' gemäss Anspruch 2, dadurch gekennzeichnet, dass man
a) 3-tert.Butyl-phenol katalytisch unter Verwendung von Raney-Nickel als Katalysator hydriert und gegebenenfalls das so erhaltene 3-tert.Butyl-cyclohexanol entsprechend verestert, oder dass man
b) 3-tert.Butyl-cyclohexanon zu einer Verbindung I' mit X = d), f) oder g) derivatisiert.

## Claims

1. An odorant composition, characterized by a content of a compound of the general formula 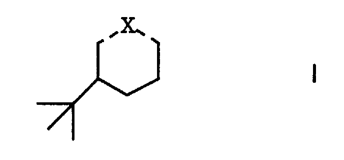 wherein ...X... has one of the meanings
a) >C=O
b) >CH-OR
wherein R is H, COCH₃, COCH₂CH₃, CO-CH(CH₃)₂, CO-CH=CH₂, COOCH₃
whereby the cis/trans ratio in the isomers of I is 65-95:35-5;
c) >C=N-OH
d) 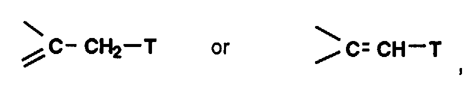 whereby T = CN or CHO
e) 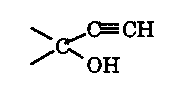
f) 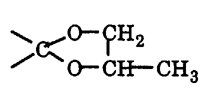
or
g)

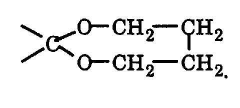

2. Compounds of the formula 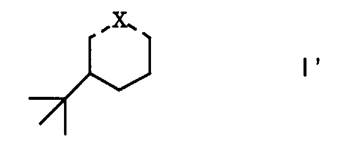 wherein ...X... has one of the meanings
b) >CH-OR
wherein R is H, COCH₃, COCH₂CH₃, CO-CH(CH₃)₂, CO-CH=CH₂, COOCH₃ ist
whereby the cis/trans ratio in the isomers of I is 65-95:35-5,
d) 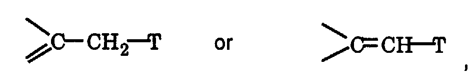 whereby T = CN or CHO
f) 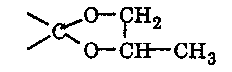
or
g)

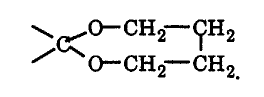

3. The use of a compound of the formula 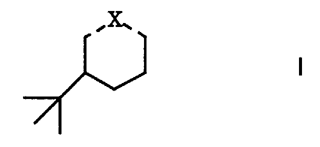 wherein ...X... has one of the meanings
a) >C=O
b) >CH-OR
wherein R is H, COCH₃, COCH₂CH₃, CO-CH(CH₃)₂, CO-CH=CH₂, COOCH₃
whereby the cis/trans ratio in the isomers of I is 65-95:35-5;
c) >C=N-OH
d) 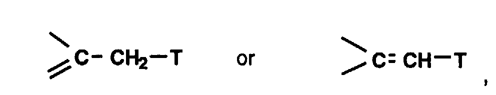 whereby T = CN or CHO
e) 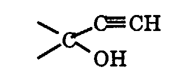
f) 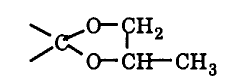
or
g) 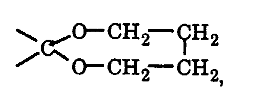 as an odorant.

4. A process for the manufacture of the compounds of formula I' in accordance with claim 2, characterized by
a) hydrogenating 3-tert.butyl-phenol catalytically using Raney-nickel as the catalyst and, if desired, correspondingly esterifying the thus-obtained 3-tert.butyl-cyclohexanol, or
b) derivatizing 3-tert.butyl-cyclohexanone to a compound I' with X = d), f) or g).

## Revendications

1. Composition odorante caractérisée en ce qu'elle contient un composé de formule générale 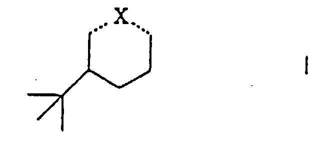 dans laquelle X représente l'un des composés
a) >C=O
b) >CH-OR
où R représente H, COCH₃, COCH₂CH₃, CC-CH(CH₃)₂,
CC-CH=CH₂, COOCH₃,
où le rapport cis/trans dans les isomères de I s'élève à 65-95:35-5,
c) >C=NOH
d) 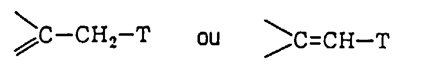 où T = CN ou CHO
e) 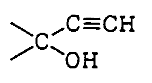
f) 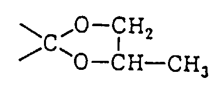 ou
g)

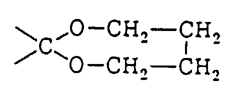

2. Composés de formule 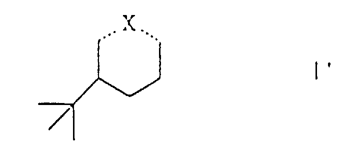 dans laquelle X a l'une des significations suivantes
b) >CH-OR
où R représente H, COCH₃, COCH₂CH₃,
CC-CH(CH₃)₂, CC-CH=CH₂, COOCH₃,
où le rapport cis/trans dans les isomères de I s'élève à 65-95-35:5,
d) 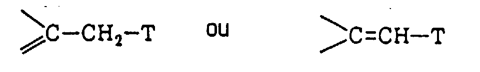 où T = CN ou CHO
f) 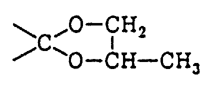
ou
g)

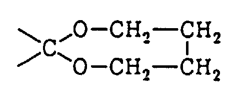

3. Utilisation d'un composé de formule 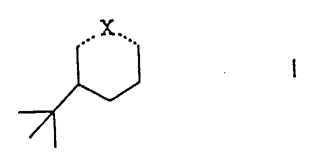 dans laquelle X a l'une des significations suivantes
a) >C=O
b) >CH-OR
où R représente H, COCH₃, COCH₂CH₃, CC-CH(CH₃)₂, CC-CH=CH₂, COOCH₃,
où le rapport cis/trans des isomères de I s'élève à 65-95:35-5,
c)>C=N-OH
d) 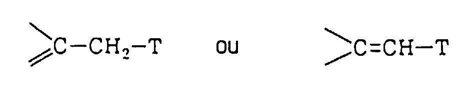 où T=CN ou CHO
e) 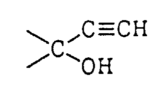
f) 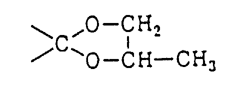
g) 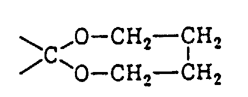 comme produit odorant.

4. Procédé de préparation des composés de formule I selon la revendication 2, caractérisé en ce que
a) on hydrogène le 3-tert.buty-phénol de façon catalytique en utilisant du nickel de Raney et le cas échéant on estérifie de façon correspondante le 3-tert.butylcyclohexanol ainsi obtenu, ou en ce que
b) on transforme la 3-tert.butyl-cyclohexanone en un composé I' dans lequel X = d), f) ou g).
